# EUROPEAN PATENT APPLICATION

(11) **EP 1 911 845 A2**
(43) Date of publication of application: **16.04.2008**
(21) Application number: 06755378.4
(22) Date of filing: 20.04.2006
(51) Int. Cl.: C12N 15/29, A01H 5/00

(54) **OCP3 GENE OF ARABIDOPSIS THALIANA AND THE OCP3 RECESSIVE MUTATION THEREOF, AND THE USE OF SAME AS A RESISTANCE REGULATOR IN PLANTS WITH DISEASES CAUSED BY NECROTROPHIC FUNGAL PATHOGENS**

(30) Priority: 22.04.2005 ES 200501035
(71) Applicant: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: COEGO GONZALEZ, Alberto, Campus Univ. Politecna de, 46022 Valencia (ES); VERA VERA, Pablo, Campus Univ. Politecna de, 46022 Valencia (ES); RAMIREZ GARCIA, Vicente Campus Univ. Politecna de, 46022 Valencia (ES); GIL MORRIO, María José Campus Unov. Politecna de, 46022 Valencia (ES)
(74) Representative: Gonzalez-Alberto Rodriguez, Natalia
(86) International application number: PCT/ES2006/070050
(87) International publication number: WO 2006/114470

(57) **Abstract**

The invention relates to the technical field of plant biotechnology and, more specifically, to the OCP3 gene of *Arabidopsis* and the *ocp3* mutation of same, as well as to the use thereof in the regulation of resistance to diseases caused by necrotrophic pathogens and to the applications of same in the generation of transgenic plants that are resistant to said type of pathogens.

SEQUENCE LIST

<110> Consejo Superior de Investigaciones Cientificas
      Universidad Politécnica de Valencia

<120> Gen OCP3

<130> P-INCI-N-05-0039

<160> 1

<170> PatentIn version 3.2

<210> 1
<211> 354
<212> PRT
<213> Arabidopsis thaliana

<400> 1

Met Ile Lys Ala Met Ala Leu Ser Ser Ala Gly Val Val Ser His Leu
1               5                   10                  15

His Pro Pro Ser Phe Ser Ser Ser Ser Gly Leu Ser Val Asn Arg Val
            20                  25                  30

Leu Phe Arg Asn Arg Asn Ala Ser Pro Cys Gly Leu Ser Leu Pro Ile
        35                  40                  45

Leu Asn Pro Ser Arg Ser Val Leu Val Phe Ala Arg Gly Lys Asn Arg
    50                  55                  60

Lys Gly Phe Val Ser Ser Ser Ser Ser Ser Pro Lys Lys Asn Lys Lys
65                  70                  75                  80

Lys Ser Leu Asp Gly Ala Asp Asn Gly Gly Gly Glu Glu Glu Glu Asp
                85                  90                  95

Pro Phe Glu Ala Leu Phe Asn Leu Leu Glu Glu Asp Leu Lys Asn Asp
            100                 105                 110

Asn Ser Asp Asp Glu Glu Ile Ser Glu Glu Glu Leu Glu Ala Leu Ala
        115                 120                 125

Asp Glu Leu Ala Arg Ala Leu Gly Val Gly Asp Asp Val Asp Asp Ile
    130                 135                 140

Asp Leu Phe Gly Ser Val Thr Gly Asp Val Asp Val Asp Val Asp Asn
145                 150                 155                 160

Asp Asp Asp Asp Asn Asp Asp Asp Asp Asn Asp Asp Asp Asp Asp Asp
                165                 170                 175

Ser Glu Glu Asp Glu Arg Pro Thr Lys Leu Lys Asn Trp Gln Leu Lys
            180                 185                 190

Arg Leu Ala Tyr Ala Leu Lys Ala Gly Arg Arg Lys Thr Ser Ile Lys
        195                 200                 205

Asn Leu Ala Ala Glu Val Cys Leu Asp Arg Ala Tyr Val Leu Glu Leu
    210                 215                 220

Leu Arg Asp Pro Pro Pro Lys Leu Leu Met Leu Ser Ala Thr Leu Pro
225                 230                 235                 240

Asp Glu Lys Pro Pro Val Ala Ala Pro Glu Asn Ser Ser Pro Asp Pro
                245                 250                 255

Ser Pro Val Glu Ser Leu Ser Ala Glu Asp Val Val Val Glu Pro Lys
            260                 265                 270

Glu Lys Val Lys Asp Glu Ala Val His Val Met Gln Gln Arg Trp Ser
        275                 280                 285

Ala Gln Lys Arg Val Lys Lys Ala His Ile Glu Thr Leu Glu Lys Val
    290                 295                 300

Tyr Arg Arg Ser Lys Arg Pro Thr Asn Ala Val Val Ser Ser Ile Val
305                 310                 315                 320

Gln Val Thr Asn Leu Pro Arg Lys Arg Val Leu Lys Trp Phe Glu Asp
                325                 330                 335

Lys Arg Ala Glu Asp Gly Val Pro Asp Lys Arg Ala Pro Tyr Gln Ala
            340                 345                 350

Pro Val

## Description

The present invention relates to the technical field of plant biotechnology and specifically to the OCP3 gene of *Arabidopsis* and *ocp3* mutation thereof, as well as to the use thereof in the regulation of resistance to diseases caused by necrotrophic pathogens, and to the applications of same in the generation of transgenic plants resistant to this type of pathogens.

### STATE OF THE ART

Plants react to phytopathogenic microorganism attacks with a series of inducible responses leading to the local and systemic expression of a broad spectrum of antimicrobial defenses. These defenses include the strengthening of mechanical barriers, oxidative burst, de novo production of antimicrobial compounds and the induction of the hypersensitive response (HR) mechanism in which the tissue surrounding the site of infection dies and in turn limits the growth of the pathogen, preventing it from spreading (Hammond-Kosack and Parker, (2003) Deciphering plant-pathogen communication: fresh perspectives for molecular resistance breeding. Curr. Op. Biotechnol. 14, 177-193)

Our understanding of how plants activate defense responses has substantially been developed, and this has been aided in part by cloning and characterization of factors of resistance to plant diseases recognizing the corresponding avirulent factors of the pathogen to induce the hypersensitive response or HR (Dangl and Jones, (2001) Plant pathogens and integrated defence responses to infection. Nature 411, 826-33)

The induction of the hypersensitive response is often associated with the development of systemic acquired resistance (SAR), another well-studied defense response providing long-lasting protection in the entire plant against a broad spectrum of pathogens (Durrant and Dong, (2004) Systemic acquired resistance. Ann. Rev. Phytopathol. 42, 185-209)

The isolation and analysis of mutants with altered defense responses (Durrant and Dong, 2004; Kunkel and Brooks, (2002) Cross talk between signaling pathways in pathogen defense. Curr. Op. Plant Biol. 5, 325-331) are aiding in the characterization of cellular components involved in signal transduction and in understanding the role of plant defense signal molecules. These studies are of vital importance for understanding the coupling of pathogen recognition to the activation of defense responses in the plant. Salicylic acid (SA), a benzoic acid derivative, is a central signal molecule central mediating different aspects of HR and SAR responses. Some time ago it was demonstrated that the synthesis and accumulation of salicylic acid is essential for creating an effective defense response against bacterial pathogens and oomycetes (Gaffney, T, Friedrich, L., Vernooij, B., Negretto, D., Nye, G, Uknes, S., Ward, E., Kessmann, H., and Ryals, J. (1993). Requirement of salicylic acid for induction of systemic acquired resistance. Science 261, 754-756). And the signaling thereof is mediated for the most part by an ankyrin repeat protein, NPR1/NIM1 (Cao, H., Glazebrook, J., Clarke, J.D., Volko, S., and Dong, X. (1997). The Arabidopsis NPR1 gene that controls systemic acquired resistance encodes a novel protein containing ankyrin repeats. Cell 88, 57-63) however, independent NPR1 pathways have been proposed and genetically identified for channeling salicylic acid signaling (Clarke, J.D., Liu, Y., Klessig, D.F., and Dong, X. (1998). Uncoupling PR gene expression from NPR1 and bacterial resistance: Characterization of the dominant Arabidopsis cpr6-1 mutant. Plant Cell 10, 557-569; Clarke, J.D., Volko, S.M., Ledford, H., Ausubel, F.M., and Dong, X (2000). Roles of Salicylic Acid, Jasmonic Acid, and Ethylene in cpr-Induced Resistance in Arabidopsis. Plant Cell 12, 2175-2190; Mayda, E., Mauch-Mani, B., and Vera, P. (2000). The Arabidopsis dth9 mutant is compromised in systemic acquired resistance without affecting SA-dependent responses. Plant Cell 12, 2119-2128; Shah, J., Kachroo, P., and Klessig, D.F. (1999). The Arabidopsis ss1 mutation restores pathogenesis-related gene expression in npr1 plants and renders defensin gene expression salicylic acid dependent. Plant Cell 11, 191-206)

In addition to salicylic acid, it has been demonstrated that other signaling molecules such as jasmonic acid (JA) and ethylene (ET), alone or in coordinated combination, regulate other aspects of the defense responses of plants (Kunkel and Brooks, 2002; Turner, J.G., Ellis, C, and Devoto, A. (2002). The jasmonate signal pathway. Plant Cell (suppl), S153-S164), and genetic indicia of this involvement in the response to fungal pathogens have also been provided. For example, certain *Arabidopsis* mutants that cannot produce jasmonic acid (for example, a triple fad3 fad7 fad8 mutant), or which cannot perceive this hormone (for example, coi1, jin1 or jar1/jin4) had altered susceptibility to different necrotrophic pathogens {Kunkel and Brooks, 2002; Lorenzo O., Chico, J.M., Sánchez-Serrano, J.J., Solano, R. (2004). JASMONATE-INSENSITIVE1 Encodes a MYC Transcription Factor Essential to Discriminate between Different Jasmonate-Regulated Defence Responses in Arabidopsis. Plant Cell 16, 1938-1950; Staswick, P.E., Yuen, G.Y., and Lehman, C.C. (1998). Jasmonate signaling mutants of Arabidopsis are susceptible to the soil fungus Pythium irregulare. Plant J. 15, 747-54; Thomma, B.P.H.J., Eggermont, K. Penninckx, I.A.M.A., Mauch-Mani, B., Vogelsang, R., Cammue, B.P.A. and Broekaert, W.F. (1998). Separate jasmonate-dependent and salicylate-dependent defense-response pathways in Arabidopsis are essential for resistance to distinct microbial pathogens. Proc. Natl. Acad. Sci. USA. 95, 15107-11; Thomma, B.P., Penninckx, I.A., Broekaert, W.F., and Cammue, B.P. (2001). The complexity of disease signaling in Arabidopsis. Curr Opin Immunol. 13, 63-68; Vijayan P, Shockey J, Levesque CA, Cook RJ., Browse J. (1998). A role for jasmonate in pathogen defense of Arabidopsis. Proc. Natl. Acad. Sci. USA. 95, 7209-7214)

Furthermore, a mutual antagonistic relationship between the signaling pathways by salicylic acid and jasmonic acid during the resistance response to diseases has been described (Kunkel and Brooks, 2002). To that respect, *Arabidopsis* mutants deficient in the accumulation of salicylic acid (SA) (for example, pad4 and eds1) or with an altered response to salicylic acid (for example, npr1) present a better induction of genes responding to jasmonic acid (JA) (Penninckx, I.A., Eggermont, K., Terras, F.R., Thomma, B.P., De Samblanx, G. W., Buchala, A., Metraux, J.P., Manners, J.M., Broekaert, W.F. (1996). Pathogen-induced systemic activation of a plant defensin gene in Arabidopsis follows a salicylic acid-independent pathway. Plant Cell 8, 2309-23; Clarke et al., 1998; Gupta, V., Willits, M.G., and Glazebrook, J. (2000). Arabidopsis thaliana EDS4 contributes to salicylic acid (SA)-dependent expression of defense responses: evidence for inhibition of jasmonic acid signaling by SA. Mol. Plant Microbe Interact. 13, 503-511)

It has been considered that the normal suppression of the response genes to JA by SA is regulated by the different cellular location of the NPR1 protein (Spoel, S.H., Koornneef, A., Claessens. S.M.C., Korzelius, J.P., van Pelt, J.A., Mueller, M.J., Buchala, A.J., Metraux, J., Brown, R., Kazan, K., Van Loon, L.C., Dong, X., and Pieterse, C.M.J. (2003). NPR1 modulates cross-talk between salicylate- and jasmonate-dependent defense pathways through a novel use in the cytosol. Plant Cell 15; 760-770)

Similarly, certain genetic studies provide indications that signaling with JA can also negatively control the expression of genes responding to SA in *Arabidopsis* (Petersen, M., Brodersen, P., Naested, H., Andreasson, E., Lindhart, U., Johansen, B., Nielsen, H.B., Lacy, M., Austin, M.J., Parker, J.E., Sharma, S.B., Klessig, D.F., Martienssen, R., Mattsson, O., Jensen, A.B., and Mundy J. (2000). Arabidopsis MAP Kinase 4 Negatively Regulates Systemic Acquired Resistance. Cell 103, 1111-1120; Kloek et al, 2001; Li, J, Brader, G., and Palva, E.T. (2004). The WRKY70 Transcription Factor: A Node of Convergence for Jasmonate-Mediated and Salicylate-Mediated Signals in Plant Defense. Plant Cell 16, 319-333)

The molecular mechanism explaining such pathway replica is not yet well understood. Therefore, the characterization of molecular components finally coordinating the signaling pathways by SA and JA is essential for understanding, and finally for creating by means of genetic engineering, very regulated resistance mechanisms providing effective protection to specific pathogen sub-series. In addition to the previously mentioned signal molecules, the production and accumulation of reactive oxygen species (ROS), mainly superoxide (O₂) and hydrogen peroxide (H₂O₂), during the course of a plant-pathogen interaction has been recognized for quite some time (Apostol, L, Heinstein, F.H., Low. P.S. (1989). Rapid stimulation of an oxidative burst during elicitation of cultured plant cells. Role in defense and signal transduction. Plant Physiol. 90, 109-116; Baker, CJ, and Orlandi, E. W. (1995). Active oxygen species in plant pathogenesis. Annu. Rev. Phytopathol. 33, 299-321)

Indicia suggest that the oxidative burst and the subsequently associated related redox signaling may have an important central role in the integration of a diverse series of defense responses of plants (Alvarez ME, Pennell RI, Meijer PJ, Ishikawa A, Dixon RA, Lamb C (1998). Reactive oxygen intermediates mediate a systemic signal network in the establishment of plant immunity. Cell 92, 773-784; Grant, J.J., and Loake, G.J. (2000). Role of reactive oxygen intermediates and cognate redox signaling in disease resistance. Plant Physiol, 124, 21-29). Furthermore, a replica between ROS- and SA- dependent defense responses has also been documented in plants (Kauss, H., Jeblick, W. (1995). Pretreatment of Parsley Suspension Cultures with Salicylic Acid Enhances Spontaneous and Elicited Production of H2O2. Plant Physiol. 108. 1171-1178; Mur, L.A., Brown, I.R., Darby, R.M., Bestwick, C.S., Bi, Y.M., Mansfield, J.W., Draper, J. (2000). A loss of resistance to avirulent bacterial pathogens in tobacco is associated with the attenuation of a salicylic acid-potentiated oxidative burst. Plant J. 23, 609-621; Shirasu, K., Nakajima, K, Rajasekhar, V.K., Dixon, R.A., and Lamb, C. (1997). Salicylic acid potentiates an agonist-dependent gain control that amplifies pathogen signals in the activation of defense mechanisms. Plant Cell. 9, 261-70; Tierens, K.F., Thomma, B.P., Bari, R.P., Garmier, M., Eggermont, K., Brouwer, M., Penninckx, I.A., Broekaert, W.F., and Cammue, B.P. (2002). Esa1, an Arabidopsis mutant with enhanced susceptibility to a range of necrotrophic fungal pathogens, shows a distorted induction of defense responses by reactive oxygen generating compounds. Plant J. 29, 131-40), but the exact mechanism and the components associating redox signaling with the induced defense response is still not well understood.

The Ep5C gene of tomato plants, which encodes a cationic peroxidase, has recently been identified and has been used as a marker for early transcription-dependent responses controlled by H₂O after the perception of a pathogen, and with a conserved gene activation mode both in tomato plants and in *Arabidopsis* plants (Coego, A., Ramirez, V., Ellul, P., Mayda, E., and Vera, P. (2005). The H2O2-regulated Ep5C gene encodes a peroxidase required for bacterial speck susceptibility in tomato. Plant J. "in press"). Since the Ep5C pathogen-induced expression is based on the production and accumulation of H₂O₂ by the affected plant cell, Ep5C is signaled as a marker for finding new defense components finally participating in the defense-related pathways in plants. For this purpose, the present invention describes the isolation and characterization of the *ocp3* mutant of *Arabidopsis thaliana* desregulated in the expression of the previously identified H₂O₂-inducible Ep5C gene. It is demonstrated that OCP3 encodes a homeobox-type transcription factor regulating different aspects of the defense response. By means of the analysis of *ocp3* mutant plants and the analysis of epistasis with other defense-related mutants, it is proposed that OCP3 controls critical aspects of the JA-mediated pathway in necrotrophic pathogens.

### DESCRIPTION OF THE INVENTION

### Isolation and characterization of the ocp3 mutant of Arabidopsis.

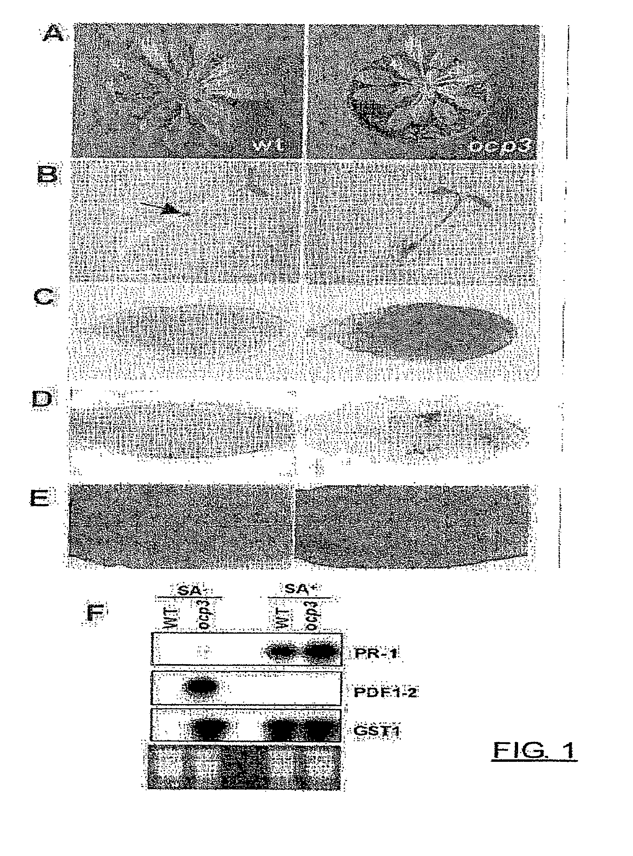

As discussed above, the Ep5C gene encodes an extracellular cationic peroxidase and is transcriptionally activated by the H₂O₂ generated during the course of plant-pathogen interactions (Coego et al., 2005). To identify signals and mechanisms involved in the induction of the Ep5C gene and testing the effect that this pathway can have on the resistance to diseases, a search was conducted for mutants using transgenic plants of *Arabidopsis* having an Ep5C-GUS gene construct (the GUS gene encodes the β-Glucuronidase enzyme). The logical basis of this research was that searching for mutants which showed constitutive expression of the indicator gene in plants cultured in non-inductive conditions, mutations which affect the regulation of this signaling pathway would be identified. Therefore, one of the transgenic lines of *Arabidopsis* Ep5C-GUS, previously characterized with ethyl methanesulfonate (EMS) was mutated and M2 plants (second generation of a mutant induced in this case by chemical or physical agents) were used to determine the existence of any constitutive expresser of GUS in the absence of any pathogenic threat. Out of approximately 10,000 M2 plants investigated, 18 constitutive expressers of GUS were identified and were left to produce seeds. The GUS activity was assayed again in the progeny of all these supposed mutants to confirm if the phenotype was inheritable. Eight lines corresponding to six complementation groups maintained the GUS constitutive activity in subsequent generations. These were called ocp (overexpression of cationic peroxidase gene promoter) mutants and the mutant selected for additional analysis was *ocp3* (Figure 1). Macroscopically, the *ocp3* plants were not very different from the wild-type plants in terms of the architecture of the plant and in terms of the growth habitat (Figure 1A). However, in early stages of plant development, the *ocp3* plants showed a delayed growth rate compared to wild-type plants. This delayed growth rate is also accompanied by the presence of a less intense green color in young leaves. Histochemical staining was carried out to investigate the expression model of the constitutive indicator gene in the *ocp3* mutants compared to non-mutated wild-type parent plants. As shown in Figure 1B, GUS activity was not detected in the parent seedlings except in a discrete area in the connection between the root and the stem (see the arrow of the left panel of Figure 1B). In contrast, GUS activity was detected in the *ocp3* seedlings in the expanding leaves as well as in the cotyledons and in the stem, but very little activity was detected in the roots. In the same way, in rosette leaves of the *ocp3* plants, GUS activity was distributed throughout the entire upper side of the leaf, whereas the leaves of the parent plants did not show detectable GUS expression detectable (Figure 1C).

As it was proposed that H₂O₂ was the signal molecule that triggered the activation of Ep5C transcription after perception of the pathogen (Coego et al., 2005), it was hypothesized that the accumulation of H₂O₂ increases in *ocp3* plants or, as an alternative, the *ocp3* mutant must be hypersensitive to ROS. To analyze if *ocp3* plants showed any phenotype related thereto, the sensitivity to H₂O₂ or to reagents directly or indirectly generating H₂O₂ was studied. *ocp3* seeds and seeds of the parent line were left to germinate in MS (Murashige and Skoog) medium which contained different amounts of H₂O₂ and the growth was recorded at different time intervals. No significant differences were found in the inhibition of growth for *ocp3* with regard to the parent seedlings. Similarly, the inhibition of growth was similar in *ocp3* and wild-type seedlings when it was assayed with light in the presence of ROS generating molecules, with Bengal rose (4,5,6,7-tetrachloro-2',9',5',7'-tetraiodofluorescein) or paraquat (methyl viologen). According to these assays, the *ocp3* mutation therefore does not confer increased sensitivity or greater resistance to oxidative stress. However, Northern blot analysis with mRNA of wild-type plants and *ocp3* plants showed (Figure 1F; lanes on the right-hand side) that the mutant seedlings constitutively expressed GST6, a gene which, as previously demonstrated, was controlled by H₂O₂ (Alvarez et al., 1998; Levine, A., Tenhaken, R., Dixon, R., and Lamb, C. (1994). H2O2 from the oxidative burst orchestrates the plant hypersensitive disease resistance response. Cell 79, 583- 93). This reflects that *ocp3* plants can be producing and/or accumulating higher levels of H₂O₂ than those that are normally found in the wild-type plants. To assay this, leaves were stained in situ with 3,3'-diaminobenzidine (DAB), a histochemical reagent which polymerizes in the presence of H₂O₂ to produce a visible reddish-brown precipitate (Thordal-Christensen, H., Zhang, Z., Wei, Y., and Collinge, D.B. (1997). Subcellular localization of H2O2 in plants. H2O2 accumulation in papillae and hypersensitive response during the barley-powdery mildew interaction. Plant J. 11, 1187-1194). Very little staining with DAB could be observed in the wild-type plant leaves (Figure 1D, left-hand side). In contrast, the *ocp3* plant leaves showed different staining foci with DAB spread out along the entire upper side of the leaf (Figure 1D, right-hand side). Furthermore, the *ocp3* plants did not show any sign of cell death or cellular collapse, as was later shown with Trypan blue staining (Figure 1E) and showed no differences compared to the wild-type when the production of superoxide anions (O₂⁻) was assayed by nitro blue tetrazolium staining.

The greatest observed accumulation of H₂O₂ and the induction of *GST6* in *ocp3* plants further suggest that the mutation produces an oxidative stress-related signal, but it does not induce a cell death response. This is consistent with the prior observation in which when H₂O₂ is generated during plant-pathogen interaction or when it is generated in situ by infiltration with different systems of generating H₂O₂, it is the signal that triggers the activation of Ep5C-GUS transcription, typical in transgenic *Arabidopsis* plants (Coego et al., 2005). Therefore, both H₂O₂ generation and the activation of the signaling mechanism leading to the activation of Ep5C transcription occur in the *ocp3* mutant.

### The ocp3 mutant has greater resistance to necrotrophic pathogens, but not to biotrophic pathogens.

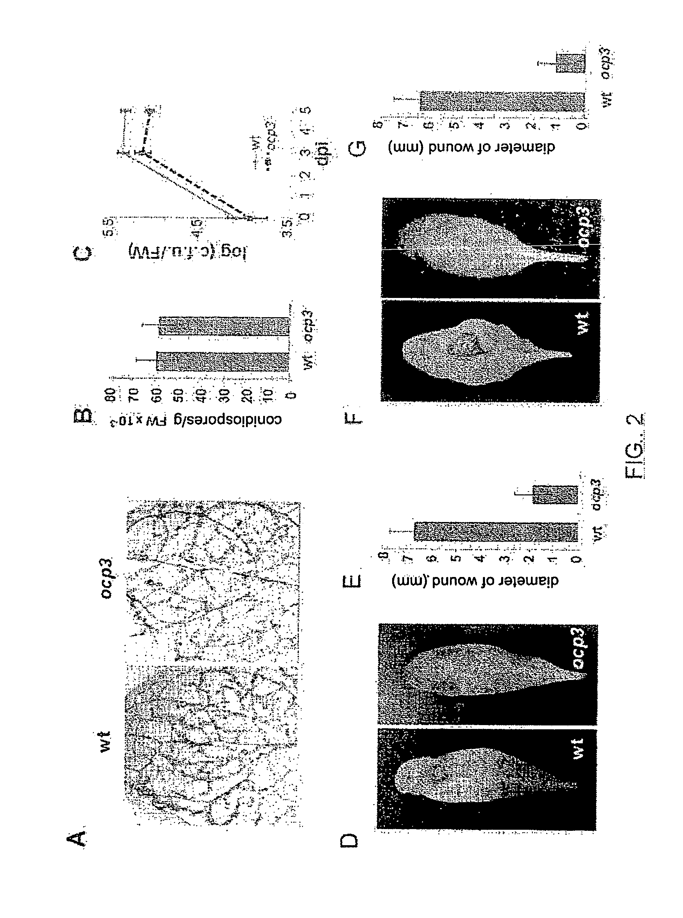

To study a causal relationship between the signaling pathway mediating the activation of Ep5C-GUS in *ocp3,* and the one mediating susceptibility to diseases, the response of this mutant to different pathogens generating diseases in *Arabidopsis* was assayed. Figure 2 shows the response of *ocp3* plants to the obliged virulent biotrophic oomycete *Peronospora parasitica* and its comparison to the response of wild-type parent plants. The growth of the pathogen was assayed by direct observation by stained hyphae in infected leaves (Figure 2A) and by the count of spores produced in the infected leaves (Figures 2B). Using these two measurements, there was no significant difference in the growth of pathogens between the wild-type plants and the *ocp3* plants. Sporulation occurred in 50% of the leaves of both wild-type plants and *ocp3* plants. Therefore, the *ocp3* mutation does not affect the susceptibility of the plant to colonization by *P. parasitica.*

Changes in the susceptibility of *ocp3* plants to pathogens were additionally studied using the virulent bacterial pathogen *Pseudomonas syringae* pv. *tomato* DC3000 (Pst DC3000) and controlling the growth rate of this bacteria in the inoculated leaves. The resulting growth curves are shown in Figure 2C. As occurs with *P. parasitica,* the growth rate of *Pst* DC3000 in *ocp3* plants was not significantly different from the growth rate observed in wild-type plants. Therefore, the susceptibility of the wild-type plant and of the *ocp3* mutant also continues virtually intact after local inoculation with this pathogen.

To determine if the *ocp3* mutation could cause changes in susceptibility to necrotrophic pathogens, plants were inoculated with *Botrytis cinerea*. The disease as evaluated between 5 and 10 days after the inoculation by tracking the degree of necrosis and death that occurred in the inoculated leaves. As was to be expected, the wild-type plants were very susceptible to *Botrytis,* and all the inoculated plants showed necrosis accompanied by extensive proliferation of the fungal mycelium (Figure 2F-G). However, and in considerable contrast, none of the *ocp3* plants which were inoculated with the same fungi showed extended necrosis in the inoculated leaves (Figure 2F-G). Furthermore, the proliferation of the fungal mycelium in *ocp3* plants was drastically inhibited. This indicates that the resistance to this necrotrophic pathogen in the *ocp3* mutant was spectacularly enhanced or the susceptibility was blocked.

To assay if the altered susceptibility to *ocp3* diseases is specific for *Botrytis,* the plants were exposed to *Plectosphaerella cucumerina,* another necrotroph. The infection of wild-type plants with *P. cucumerina* also led to a strong degradation of the tissue of the leaf, manifested as extended wounds and chlorosis which increases in diameter as the infection progresses throughout the inoculated leaf (Figure 2D). In contrast, the *ocp3* plants showed a high degree of resistance to this fungal pathogen, since the visible and measurable necrosis of the inoculated leaves was drastically reduced (Figure 2D-E), and the proliferation of the fungal mycelium was also drastically inhibited.

Based on these results, it can be concluded that the susceptibility to necrotrophic fungi is a typical feature associated with the OCP3 locus, and the mutation identified in this locus causes greater resistance to the same pathogens. This consideration is also consistent with the observation that PDF1.2, a marker gene the expression of which is inducible for the response to ET/JA defense pathway against necrotrophic fungal pathogens (Turner et al, 2002), is constitutively expressed in *ocp3* plants (Figure 1F).

### The greater resistance of ocp3 plants to necrotrophic fungi requires jasmonic acid (JA) but not salicylic acid (SA) or ethylene (ET)

The constitutive expression in *ocp3* plants of the *GST6* gene inducible by H₂O₂ and of the PDF1.2 gene by JA, but not of the *PR-1* gene inducible by SA, (Figure 1F), suggests an association between oxidative stress and signaling with JA, which is apparently independent of SA. In the complex scheme of interactions taking place during the resistance responses of plants, an antagonistic relationship between the SA and JA/ET pathway has been documented for some time (Kunkel and Brooks, 2002) and indicates that the constitutive activation of the pathway leading to the expression of the PDF1.2 gene in *ocp3* plants could be the negation of the expression of SA-dependent genes. However, the exogenous application of SA promotes the expression of the *PR-1* marker gene both in *ocp3* plants and in wild-type plants (Figure 1F), indicating that the *ocp3* plants are not affected in SA perception, and coincides with the observation that resistance response to biotrophic pathogens is also intact in this mutant (Figure 2A-C). The exogenous application of SA furthermore annuls the constitutive expression of PDF1.2 taking place in *ocp3* plants (Figure 1F). This antagonistic effect of SA was specific for the expression of PDF1.2, since the expression of *GST6* was not repressed in *ocp3* after the treatment with SA. Instead, SA promoted the activation of GST1 in wild-type plants (Figure 1F). This last observation reinforces the association existing between SA and ROS as previously documented by other authors (Mur et al, 2000; Shirasu et al, 1997; Tierens et al, 2002), but it also indicates that the oxidative stress mediating the expression of GST1 in *ocp3* plants and the joint expression of PDF1.2 could be independent of SA.

To more directly evaluate if SA could contribute to the *ocp3* plant phenotype in relation to the observed resistance to necrotrophic pathogens, the nahG transgene is crossed in ocp3. nahG encodes a salicylate hydroxylase blocking the SA pathway by means of SA degradation (Delaney, T.P., Uknes, S., Vernooij, S., Friedrich, L., Weymann, K., Negrotto, D., Gaffney, T., Gutrella, M., Kessmann, H., Ward, E., and Ryals, J. (1994). A central role of salicylic acid in plant disease resistance. Science, 266, 1247-1250).

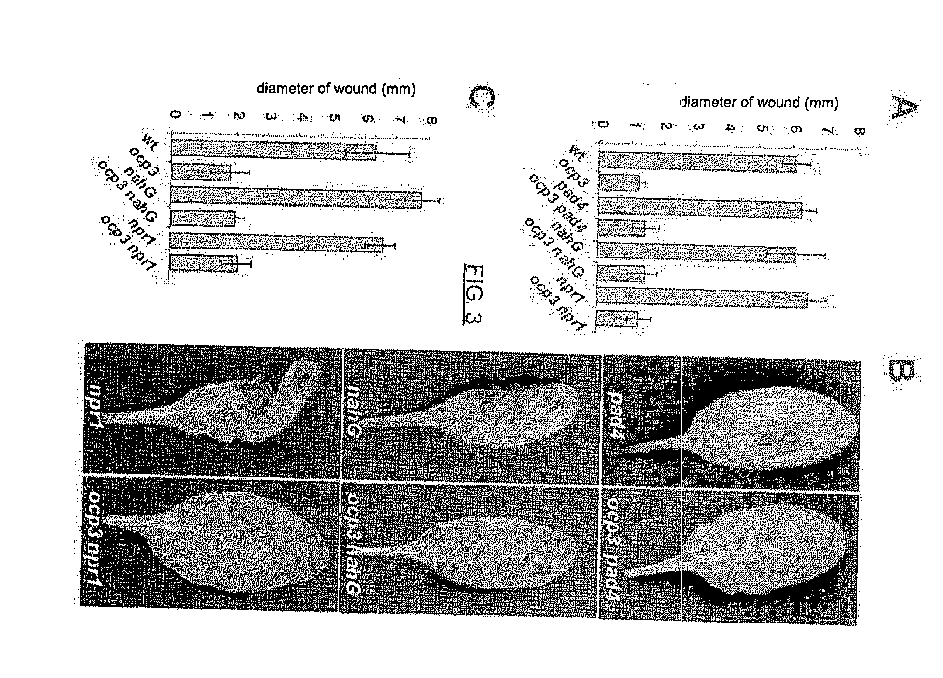

The *ocp3 nahG* plants retained the resistance to infections by *B. cinerea* (Figure 3A-B) and *P. cucumerina* (Figure 3C) at levels similar to those of the *ocp3* plants. Similarly, when pad4 plants, which are also affected in the accumulation of SA after the attack by pathogens (Zhou, N, Tootle, T.L., Tsui, F., Klessig, D.F., and Glazebrook, J. (1998). PAD4 functions upstream from salicylic acid to control defense responses in Arabidopsis. Plant Cell 10, 1021-1030), were subjected to introgression in *ocp3* plants, the resulting *ocp3 pad4* plants remained as resistant to *B. cinerea* (Figure 3A-B) or to *P. cucumerina* (Figure 3C) as the *ocp3* plants.

A double *ocp3 npr1-1* mutant was created to additionally broaden these studies. The *npr1-1* mutant was originally identified by its insensitivity to SA and is currently considered the main regulator of SA-mediated responses (Durrant and Dong, 2004). As is observed for *ocp3 nahG* and *ocp3 pad4* plants, the resistance of *ocp3 npr1-1* plants to necrotrophic fungi also remained unchanged with regard to that observed in *ocp3* plants (Figure 3A-C). All these results therefore indicate that it seems that SA is not required to improve the resistance to necrotrophic pathogens that can be attributed to the *ocp3* mutation.

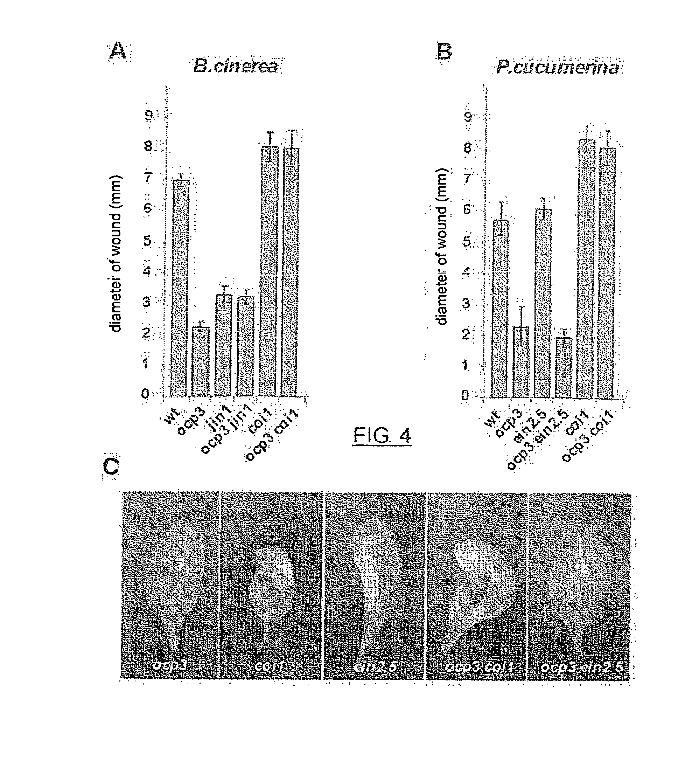

The importance of JA in the contribution to the *ocp3* plant phenotype was evaluated as an alternative to SA. It was assayed if a defect in the perception of this hormone may affect the better observed resistance of *ocp3* plants to necrotrophic fungi. The coi1 mutant of *Arabidopsis* is completely insensitive to JA and the COI1 protein is required for all JA-dependent responses identified up until now. COI1 encodes an F-box protein involved in the ubiquitin-mediated degradation of the signaling by JA by means of the formation of functional E3-type ubiquitin ligase complexes (Xie, D.X., Feys, B.F., James, S., Nieto-Rostro, M., and Turner, J.G. (1998). COI1: An Arabidopsis gene required for jasmonate-regulated defense and fertility. Science 280, 1091-1094; Devoto, A., Nieto-Rostro, M., Xie, D., Ellis, C, Harmston, R., Patrick, E., Davis, J., Sharratt, L., Coleman, M., and Turner, J.G. (2002). COI1 links jasmonate signalling and fertility to the SCF ubiquitin-ligase complex in Arabidopsis. Plant J. 32, 457-466). Furthermore, the coi1 plants cannot express PDF1.2 and show greater sensitivity to necrotrophic fungi (Thomma et al., 1998; Turner et al. 2002). All this indicates the importance of JA in the resistance of plants to this type of pathogens and justifies the introgression of coi1 in *ocp3* to generate double *ocp3 coi1* mutant plants (Figure 4). The greater resistance observed in *ocp3* plants to *B. cinerea* and P. *cucumerina* is significantly annulled when the *coi1* mutation is present (Figure 4 A-C). The *ocp3 coi1* plants behave like *coi1* plants which are very affected after infection with any fungus, the necrotic wounds extending throughout the inoculated leaves as shown in Figure 4C for the response to *P. cucumerina.* The study of *jin1*, another mutant insensitive to JA was considered in addition to coi1 (Berger, S., Bell, E., and Mullet, J.E. (1996). Two Methyl Jasmonate-Insensitive Mutants Show Altered Expression of AtVsp in Response to Methyl Jasmonate and Wounding. Plant Physiol. 111, 525-531) in relation to the *ocp3* mutant. JIN1 is a bHLHzip-type MYC-type transcription factor which functions dependently on COI1 (Lorenzo et al., 2004). Unlike that which occurs with coi1 and despite the defect in signaling with JA, the jin1 plants show greater resistance to necrotrophic pathogens, indicating that JIN1 can function as a repressor of the resistance to this type of pathogens. Curiously enough, double *ocp3 jin1* mutant plants remained very resistant when they were assayed against infection by *B. cinerea* (Figure 4A) and at levels comparable to those obtained by *jin1* or *ocp3* plants. It must be mentioned that the *ocp3* mutation does not confer sensitivity to JA (according to the assay for inhibiting the growth of roots in the presence of JA) nor is it allelic to jin1. This indicates that there may be certain redundancy or overlapping of functions for the two mutants under consideration to improve the resistance to B. *cinerea* that is finally induced by JA and controlled by COI1.

It has also been demonstrated that ethylene (ET) mediates certain aspects of the responses of the plants to pathogens (Berrocal-Lobo, M., Molina, A., and Solano, R. (2002). Constitutive expression of ETHYLENE-RESPONSE-FACTOR1 in Arabidopsis confers resistance to several necrotrophic fungi. Plant J. 29, 23-32; Thomma et al., 2001). However, signaling with ET can also function independently of JA, or it can even inhibit JA-dependent responses (Ellis, C. and Turner, J.G. (2001). The Arabidopsis mutant cev1 has constitutively active jasmonate and ethylene signal pathways and enhanced resistance to pathogens. Plant Cell, 13, 1025-1033; Thomma et al, 2001). *ocp3* plants were crossed with the *ein2* mutant insensitive to ET to assay the importance of ET in the *ocp3* mutation-mediated resistance response (Alonso, J.M., Hirayama, T., Roman, G., Nourizadeh, S. and Ecker, J.R. (1999) EIN2, a bifunctional transducer of ethylene and stress responses in Arabidopsis. Science, 284, 2148-2152) to generate the double *ocp3 ein2* mutant. As can be seen in Figure 2B-C, the resistance of *ocp3 ein2* plants to P. *cucumerina* remained unchanged compared to the resistance observed in *ocp3* plants (Figure 4A-C), thus indicating that the ET plant hormone is essential for the observed *ocp3*-mediated resistance.

### Isolation of OCP3

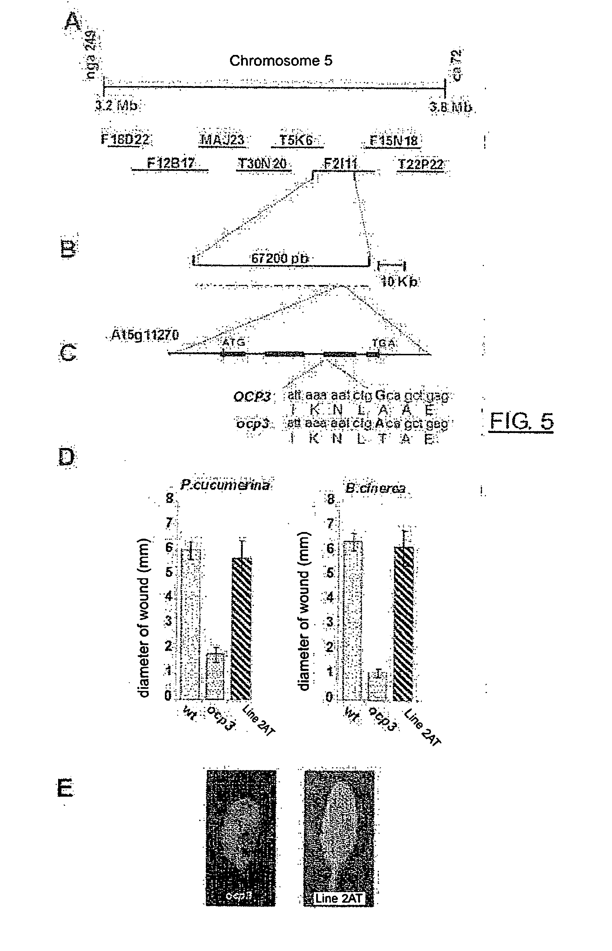

Wild-type *ocp3*/*ocp3* plants and OCP3/OCP3 plants that contained the Ep5C-GUS transgene were backcrossed and the progeny was analyzed to determine the nature of the mutation. The constitutive expression of GUS activity in the 21 assayed seedlings was absent in the F1 plants resulting from this crossing, and the expression was present in 31 of 118 seedlings in the F2 plants. The F2 segregation ratio of the phenotype conferred by *ocp3* was 1:3 (constitutive expressers:non-expressers, α² = 1.48 (0.1 > P > 0.5)), indicating a single recessive mutation. The *ocp3* mutant was subjected to backcrossing with wild-type *Landsberg erecta* (Ler) to generate an F2 mapping population and the recombinant seedlings were identified by means of the use of single sequence length polymorphism (SSLP) markers (Bell, CJ., and Ecker, J.R. (1994). Assignment of 30 microsatellite loci to the linkage map of Arabidopsis. Genomics 19, 137-144). DNA was initially isolated from 38 homozygotic *ocp3* plants and the segregation of the SSLP markers indicated that *ocp3* showed an association to the Nga249 marker in chromosome 5, in which all of the 76 alleles analyzed were Co1-0 alleles (0 *erecta:* 76 Co1-0). Another analysis of the *ocp3* plants screened with additional markers available for chromosome 5 identified the SSLP, Nga249 and ca72 markers as the closest markers that flanked the *ocp3* mutation on each side (Figure 5A). The screening of 1100 plants randomly chosen from an F2 Ler x *ocp3* mapping population with the SSLP, Nga249 and ca72 markers identified 29 plants which had a recombination in the interval. By using these 29 recombinant plants it could be seen that OCP3 was located at 4 cM from Nga249 and at 1.9 cM from ca72. Other polymorphic markers were designed for the region comprised between Nga249 and ca72 and the position of OCP3 narrowed to a genome region which included the end of the bacterial artificial clone (BAC) T5KB and the start of the BAC clone F2I11. 19 genes are present in the mentioned sequence comprised within these two BAC clones (Figure 5B). The entire coding region of each of these genes in *ocp3* plants was amplified and the PCR product sequences were determined. The sequence corresponding to the At5g11270 gene was identified as the only sequence that showed a single nucleotide substitution (G-to-A in the coding chain (exon 3)) that caused a single amino acid substitution (from Ala to Thr) (Figure 5C). No mutation was found in the remaining eighteen genes. At5g11270 contains two introns and encodes a protein with 553 amino acids.

A 3.2 Kb fragment containing At5g11270 in *ocp3* was introduced by the Agrobacterium-mediated transformation to unequivocally assign At5g11270 as OCP3. Three transgenic lines were assayed with regard to the constitutive expression of GUS and with regard to the resistance to B. *cinerea* and P. *cucumerina.* In all these lines, the constitutive expression of GUS was annulled and normal susceptibility to the fungal pathogens had been recovered, demonstrating that At5g11270 is OCP3 (Figure 5D-E summarizes the result of this complementation for one of the transgenic lines generated, line 2AT).

### The expression of OCP3 is partially repressed by fungal infection

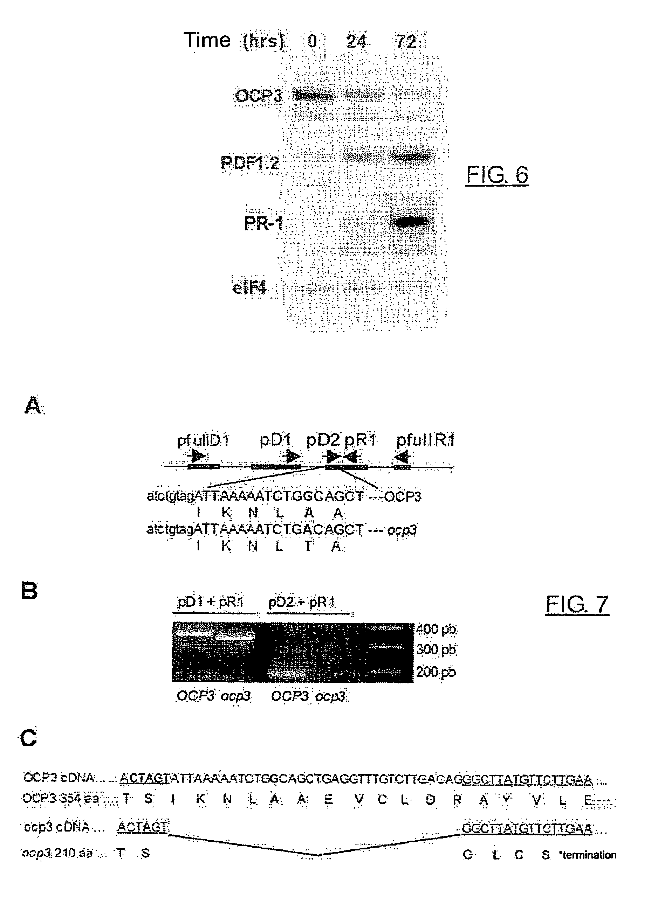

The expression of OCP3 in response to infection with a necrotrophic fungal pathogen in wild-type plants at different time intervals after the infection was analyzed. Levels of OCP3 mRNA could not be detected by Northern blot analysis in any analyzed tissue, indicating that the OCP3 gene is transcribed at a very low rate. To solve this problem, the presence of OCP3 mRNA was studied by RT-PCR. These analyses showed that OCP3 is constitutively expressed in healthy plant leaf tissue. Figure 6 demonstrates that after infection with P. *cucumerina* there is a reduction in the level of accumulation of OCP3 mRNA, being more evident 72 hours after infection. Together with this reduction, and inversely correlated, the marker gene inducible by JA and by PDF1.2 fungi is positively regulated after infection with P. *cucumerina.* An induced expression of the defense-related PR1 gene takes place in post-infection steps and is indicative of the deterioration of tissues which occurs as a result of the growth habitat of the fungus.

The negative regulation of OCP3 after fungal infection, its inverse correlation with the induced expression of PDF1.2 and the recessive nature of the *ocp3* mutation favors the interpretation that OCP3 functions as a repressor of the resistance response to fungal pathogens in wild-type plants.

### Aberrant processing of At5g11270 mRNA in the ocp3 mutant

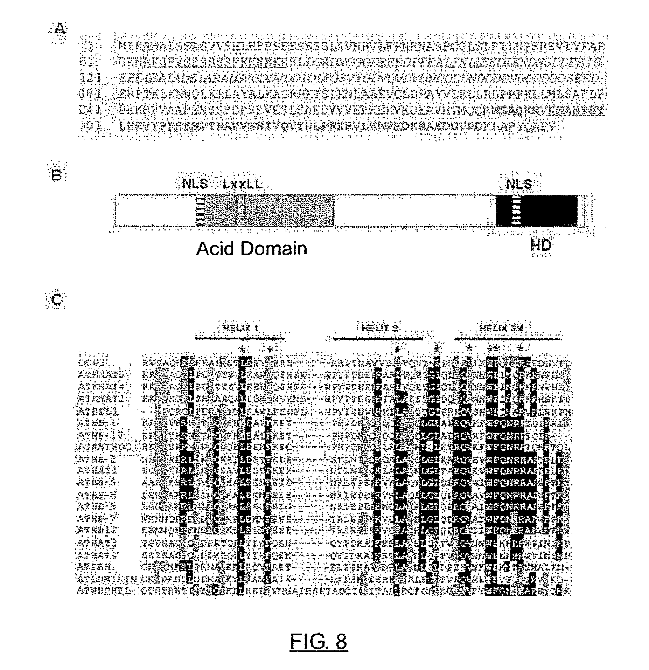

A 1.2 Kb fragment was amplified by reverse transcription-mediated polymerase chain reaction (RT-PCR) from wild-type and *ocp3* mutant plants to identify the structure of the OCP3 gene and its *ocp3* mutant allele, using primers designed according to the mentioned sequence of the At5g11270 gene. Direct sequencing and comparison of the RT-PCR products showed that the cDNA derived from *ocp3* has an internal deletion of 36 nucleotides instead of the expected substitution of a single nucleotide, identified in the genome sequences (Figure 7). This deletion corresponds to the first 36 nucleotides of exon III. The G-to-A transition identified at the genome level in the encoding chain of the *ocp3* allele (Figure 5C) thus causes an alteration in the normal processing for the mRNA derived from *ocp3.* This short deletion causes a phase shift in the open reading frame of *ocp3* resulting in the generation of an in-phase termination codon causing a truncated protein with 210 amino acid residues instead of the 354 residues of the wild-type protein (see below, Figure 8). This deletion was additionally confirmed in different *ocp3* plants by RT-PCR using a series of internal primers designed from the genome sequence (Figure 7A). Products having expected lengths were obtained in all the reactions except when the internal primer in the deleted sequence was used, which did not result in any RT-PCR product in samples derived from *ocp3* plants (Figure 7B). The lack of use genetically attributed to the *ocp3* recessive mutation therefore is not due to a change of amino acid due to the single substitution of nucleotides observed in the genome sequence; instead it is based on a abnormal processing of the mRNA transcribed from the mutated *ocp3* version which, after translation, produces a truncated protein lacking 144 amino acid residues of the C-terminal part (Figure 7 and 8).

### OCP3 encodes a homeobox transcription factor

DNA sequencing demonstrated that OCP3 cDNA encodes a protein having 354 amino acid residues (Figure 8A) of 39111 D and a pI of 4.53. OCP3 contains different detectable characteristics. A 60 amino acid domain (position 284 to 344) resembling that of a homeodomain (HD) encoded by homeobox genes of different organisms is identified close to the C-terminal end (Gehring, W.J., Affolter, M. and Bürglin, T. (1994). Homeodomain proteins. Annu Rev Biochem, 63, 487-526). The homeodomain of OCP3 shares the majority of the very conserved amino acids forming the typical signature of the 60 amino acid HD module. The conservation of these critical residues (for example, L-16, Y-20 instead of F-20, I/L-34, I/L/M-40, W-48, F-49 and R-53) is easily identified in comparison with different proteins containing homeodomains of *Arabidopsis* which belong to different subgroups of proteins (Figure 8C). The inspection of amino acid sequence of OCP3 also showed the presence of two canonic bipartite nuclear localization signals (NLS) (Dingwall, C, and Laskey, R.A. (1991). Nuclear targeting sequences-a consensus? Trends Biochem. Sci. 16, 478-481; Nigg, E.A. (1997). Nucleocytoplasmic transport: signals, mechanism and regulation. Nature 386: 779- 787), RK-(X)₁₀-KKNKKK in positions 64-81, and KK-(X)₁₀-RRSKR in positions 294-310, the latter being hidden within the homeodomain (Figure 8A). These characteristics could be mediating a direction of the protein to the nucleus. Another detectable characteristic of OCP3 is the presence of an extended region rich in acid residues (positions 84-181), a common characteristic of several transcription activators (Cress, W.D., and S.J. Triezenberg. 1991. Critical structural elements of the VP16 transcriptional activation domain. Science 251: 87-90). The last identifiable characteristic within OCP3 is the presence of the canonic LxxLL motif in positions 101-105 (Figure 8A). This motif is a typical sequence aiding the interaction of different transcription co-activators with nuclear receptors, and it is thus a defining characteristic identified in several nuclear proteins (Heery, D.M., E, Kalkhoven, S. Hoare, and M.G. Parker. 1997. A signature motif in transcriptional co-activators mediates binding to nuclear receptors. Nature 387: 733-736). All these structural motifs strongly indicate that OCP3 is a nuclear protein involved in the regulation of transcription in *Arabidopsis.* According to a general classification scheme for homeobox genes (http://www.homeobox.cjb.net/) OCP3 is unique since it is different from the main classes of proteins containing homeodomains found in plants, including KNOX or HD-ZIP. OCP3 is furthermore present as a gene of a single copy in the genome of *Arabidopsis.* The searches for sequences in databases showed an extensive identity of OCP3 with six other proteins - tomato protein (GenBank accession number AW223899, 48.9% identity), potato protein (GenBank accession number BQ112211, 48.3% identity), grape protein (GenBank accession number CD003732, 51.5% identity), rice protein (GenBank accession number AY224485, 49.5% identity), wheat protein (GenBAnk accession number CK205563, 49.4% identity) and corn protein (GenBAnk accession number BG840814, 51.3% identity) - which, as was seen, had a high degree of sequence similarity with OCP3 and with the conservation of the main structural motifs previously shown. This indicates that the use of this type of transcription regulator has been well conserved in plants throughout evolution.

The data set forth in the present invention provides evidence of a role of OCP3 in the regulation of resistance to necrotrophic pathogen microorganisms. A recessive mutation in the OCP3 gene resulted in a greater resistance of *ocp3* plants to the fungal pathogens *Botrytis cinerea* and *Plectosphaerella cucumerina,* whereas the resistance to the infection by the oomycete *Peronospora parasitica* or the bacterium *Pseudomonas syringae DC3000* remained invariable in the same plants. Interestingly enough, the OCP3 gene was expressed in very low levels in healthy plants and this constitutive expression is partially repressed during the infection with a fungal necrotroph. The resistance phenotype conferred by the *ocp3* mutation is furthermore blocked when the assay is carried out in the coi1 mutant as a base organism, the double *ocp3 coi1* mutant plants retaining the greater sensitivity to the necrotrophs that can be attributed to coi1. This means that OCP3 participates in the defense response regulated by JA. In fact, the *ocp3* recessive mutation confers constitutive expression of the PDF1.2 gene, encoding a defense protein with a defined role in the JA-mediated defense response of the plants (Thomma et al., 1998). Since the expression of PDF1.2 is completely dependent on COI1 (Turner et al., 2002) and is in healthy mutant *ocp3* plants, the consideration that OCP3 is functioning in a COI1-dependant manner, acting as a negative regulator of the JA-mediated defense response to necrotrophic pathogens, is reinforced. Another important feature of *ocp3* is the greater accumulation of H₂O₂ which is observed to occur in rest conditions in the leaves of *ocp3* plants, followed by H₂O₂-inducible marker gene GST1 (Levine et al., 1994; Alvarez et al., 1998) but without symptoms indicating cell death. H₂O₂ and other ROI molecules are normally produced in high levels during the infection by both biotrophic pathogens and necrotrophic pathogens and have been involved as regulating signals for the baseline resistance response to these pathogens (Mengiste, T, Chen, X., Salmerón, J.M., and Dietrich, R.A. (2003). The BOS1 gene encodes an R2R3MYB transcription factor protein that is required for biotic and abiotic stress responses in Arabidopsis. Plant Cell 15, 2551-2565; Tiedemann, A. V. (1997). Evidence for a primary role of active oxygen species in induction of host cell death during infection of bean leaves with Botrytis cinerea. Physiol. Mol. Plant Pathol. 50, 151-166) However, out of the pathogens assayed in ocp3 plants, an increase of the resistance was only observed towards necrotrophic pathogens, whereas the resistance to biotrophic pathogens remained intact. This significant difference indicates that the *ocp3* mutation can affect specific functions related to ROI by means of the regulation of certain effector molecules aimed at the sensitization and identification of a necrotroph. Interestingly enough, it has been shown that SA and H₂O₂ form a circuit which is a feedback loop during the course of a plant-pathogen interaction (Shirasu et al., 1997; Draper, J. (1997). Salicylate, superoxide synthesis and cell suicide in plant defence. Trends Plant Sci. 2, 162-165), and there are indications suggesting that SA can be necessary for a local response local to a necrotroph such as *Botrytis* at the infection point (Govrin, E.M., and Levine, A. (2000). The hypersensitive response facilitates plant infection by the necrotrophic pathogen Botrytis cinerea. Curr. Biol. 10, 751-757; Ferrari, S., Plotnikova, J.M., De Lorenzo, G., and Ausubel, FM. (2003). Arabidopsis local resistance to Botrytis cinerea involves salicylic acid and camalexin and requires EDS4 and PAD2, but not SID2, EDS5 or PAD4. Plant J. 35, 193-205).

However, the synthesis and accumulation of SA is neither increased nor repressed in *ocp3* plants. Furthermore, the analysis of double mutant plants for *ocp3* and key regulators of the accumulation and perception of SA, such as the double mutants *ocp3 pad4, ocp3 nahG or ocp3 npr1* generated in the present invention, indicate that SA is not required for *ocp3-*mediated resistance to necrotrophs. Furthermore, the plant hormone ethylene (ET) does not seem to be necessary for achieving the greatest resistance of *ocp3.* The lack of perception of this hormone, as studied with the double mutant *ocp3 ein2* plants, does not here repress or reduce the characteristics resistance of the ocp3 plants to *P. cucumerina.* Since JA and ET can function in association or independently for the activation of specific signaling pathways (Thomma et al., 2001, Ellis and Turner, 2001), the independent resistance of ET of ocp3 indicates that OCP3 regulates a specific branch of the JA pathway. This branch further seems to be independent of another branch which has been proposed to be controlled by the transcription factor JIN1 regulated by JA (Lorenzo et al., 2004), because epistasis between *ocp3* and *jin1* was not found at least when the resistance to *B. cinerea* was assayed. All these observations thus confirm the important role of OCP3 in the specific regulation of a COI1-dependant resistance to necrotrophic pathogens. OCP3 is a member of the homeobox gene family. Homeobox proteins are ubiquitous in higher organisms and represent master control changes involved in development processes and in cell adaptation to changes in the medium. They function as transcription regulators which are characterized by the presence of an evolutionally conserved homeodomain (HD) responsible for the specific binding to DNA (Gehring et al., 1994). Two main classes of genes encoding HD have been identified in plants: the HD class represented by KNOTTED1 (Vollbrecht, E., Veit, S., Sinha, N. and Hake, S. (1991). The developmental gene Knotted-1 is a member of a maize homeobox gene family. Nature 350, 241-243) and the HD-Zip protein family (Schena, M., and Davis, R. W. (1992). HD-Zip proteins: members of an Arabidopsis homeodomain superfamily. Proc Natl Acad Sd USA, 89, 3894-3898) The latter is characterized by an additional leucine zip motif, adjacent to the HD facilitating the homo- and heterodimerization of transcription regulators. The functional characterization of some members of the homeobox family confirms a role, for some of them, as key regulators of the signaling with hormones (Himmelbach, A., Hoffmann, T., Laube, M., Hohener. B., and Grill, E. (2002). Homeodomain protein ATHB6 is a target of the ABI1 protein phosphatase and regulates hormone responses in Arabidopsis. EMBO J 21: 3029-3038), in adaptation responses to environmental parameters (Steindler, C., Matteucci, A., Sessa,G., Weimar, T., Ohgishi, M., Aoyama, T., Morelli, G. and Ruberti I. (1999). Shade avoidance responses are mediated by the ATHB-2 HD-Zip protein, a negative regulator of gene expression. Development, 126, 4235-4245. ; Zhu, J., Shi, H., Lee, B-h., Damsz, B., Cheng, S., Stirm, V., Zhu J-K., Hasegawa, P., Bressan, R.A. (2004). An Arabidopsis homeodomain transcription factor gene, HOS9, mediates cold tolerance through a CBF-independent pathway. Proc. Natl. Acad. Sci USA 101, 9873-9878) and in pathogen-derived signaling processes (Mayda, E., Tornero., P., Conejero, V., and Vera, P. (1999). A tomato homeobox gene (HD-Zip) is involved in limiting the spread of programmed cell death. Plant J. 20, 591-600).

The isolated mutation identified in ocp3 results in an abnormal processing of the corresponding transcript causing an internal deletion where the first 36 nucleotides of exon III are no longer present in mature ocp3 mRNA. This short deletion causes a phase shift in the ORF of ocp3 resulting in the generation of a premature termination codon. This mutation thus produces a truncated ocp3 protein consisting of 210 amino acid residues instead of the 354 amino acid residues characteristic of OCP3. The expected 10 amino acid domain corresponding to the homeodomain (HD) is located within the 144 amino acid C-terminal domain missing in *ocp3.* It is conceivable that the mutated ocp3 protein no longer functions as a transcription regulator since this domain is a determining factor for the homeobox proteins to function as transcription regulators, because this is the site where contact with DNA is established, mainly through helix 3 of the HD domain, which is directed to the main groove of the DNA helix present in the downstream gene promoting region (Gehring et al. 1994). Therefore, OCP3 functions as a specific transcription factor of the COI1-dependant, JA-mediated plant cell signal translation pathway and modulates the transcription of important genes for the defense response or responses to necrotrophic pathogens.

The identification of target OCP3 genes and the interaction with molecules of associated proteins is the challenge for the future. Furthermore, the possible interaction of OCP3 with other transcription regulators involved in the defense response to necrotrophic pathogens such as the JIN1 protein related to MYC {Lorenzo et al., 2004), the AP2-type ERF1 protein (Lorenzo, O., Piqueras, R., Sánchez-Serrano, J. J., and Solano, R. (2003). ETHYLENE RESPONSE FACTOR1 integrates signals from ethylene and jasmonate pathways in plant defense. Plant Cell 15, 165-178), the BOS1 protein related to MYB (Mengiste et al., 2003) or the transcription factor WRKY70 (Li et al., 2004), and how they function in a coordinated manner is another interesting challenge for the future. All these strategies must aid in understanding the regulating mechanism of the use of OCP3 and how this use can be exploited to generate plants that are more resistant to fungal pathogens without affecting the defense responses against other types of pathogens. The genetic manipulation of the OCP3 gene by means mechanisms of reverse genetics in transgenic plants repressing or altering the gene expression of OCP3, or altering its function as a transcription factor, will allow the agronomic exploitation of the use of OCP3 in the regulation of the defensive response of the plants against said pathogenic aggressions.

### DESCRIPTION OF THE DRAWINGS

### Figure 1. Characterization of ocp3 plants and comparison with wild-type plants.

(A) A comparison of the external appearance of *ocp3* plants (at the right-hand side) and the wild-type parent plants (wt) (at the left-hand side). The plants were photographed when they were 3.5 weeks old.
(B) Histochemical staining of GUS activity directed by the Ep5C promoter in a 10-day-old wild-type transgenic seedling (at the left-hand side) and seedlings *ocp3* (at the right-hand side) cultivated in MS agar medium. The arrow indicates a discrete tissue area in the connection of the hypocotyl and the root where GUS activity is observed in the wild-type seedlings.
(C) Completely expanded rosette leaves of transgenic wild-type plants (at the left-hand side) and of *ocp3* plants (at the right-hand side), stained to determine the GUS activity.
(D) H₂O₂ production in wild-type plants (at the left-hand side) and *ocp3* plants (at the right-hand side). H₂O₂ production was assayed using 3,3'-diaminobenzidine. The reddish-brown color indicates the polymerization of 3,3'-diaminobenzidine in the H₂O₂ production site.
(E) Staining of leaf tissue of wild-type plants (at the left-hand side) and *ocp3* plants (at the right-hand side) with Trypan blue in the search for signs of cell death. The absence of cell collapse is revealed by the lack of intense blue spots after staining with Trypan blue.
(F) Expression of genes markers PR-1, PDF1.2 and GST1 in wild-type and *ocp3* plants 36 hours after the plants have been sprayed with (+SA) or without (-SA) a buffer solution containing 0.3 mM salicylic acid (SA).

### Figure 2. Resistance of ocp3 plants to necrotrophic pathogens but not to biotrophic pathogens.

(A) Resistance response of wild-type *Arabidopsis* plants and *ocp3* mutants to virulent *Peronospora parasitica.* Seven days after the inoculation by spraying 2-week-old plants with 10⁵ conidiospores per milliliter of *Peronospora,* the leaves were stained with lactophenol-Trypan blue and were viewed under a microscope to reveal the extensive growth characteristics of the hyphae and the conidiospores.
(B) To quantify the resistance to *P. parasitica,* conidiospore production was quantified seven days after the inoculation with the aid of a hemocytometer. The plants carrying the *ocp3* mutation were as resistant to this pathogen as the wild-type plants.
(C) Growth of *Pseudomonas syringae* pv. tomato DC3000 in *ocp3* and wild-type plants. A bacterial suspension was infiltrated in 4-week-old plants, and the bacterial titer, measured as c.f.u. by fresh weight, was determined 0, 3 and 5 days after the infection in wild-type plants (solid lines) and *ocp3* (dotted lines) The error bars show the 95% confidence limits of the data transformed into logarithms. 8 samples were taken for each genotype at each point in time. The experiment was repeated 3 times, similar results being obtained.
(D) Representative leaves of *ocp3* and wild-type plants 4 days after the inoculation with a drop of 6 µl of a spore suspension (5 x 10⁶ spores/ml) of P. *cucumerina.*
(E) The symptoms of the disease measured as the size of the wound were evaluated 6 days after the inoculation with P. *cucumerina*, determining the mean diameters of the wound in 3 leaves of 8 plants. The data points show the mean size of the wound ± SE of the measurements.
(F) Representative leaves of *ocp3* and wild-type plants 4 days after the inoculation with a drop of 6 µl of spores of *Botrytis cinerea* (2,5 x 10⁴ conidia/ml).
(G) The size of the wound generated by *Botrytis cinerea* was measured 6 days after the inoculation. The data points show the mean size of the wound ± SE of the measurements for at least 30 wounds.

All the experiments were repeated at least 3 times with similar results, wt, wild-type.

### Figure 3. Effect of mutations related to SA on the resistance response to diseases of ocp3 plants

(A) Resistance response of *ocp3 nahG, ocp3 npr1* and *ocp3 pad4* double mutants to *Botrytis cinerea* compared to that of simple mutant genotype and wild-type plants. The plants were inoculated and the symptoms of the disease were evaluated as described in Figure 2, determining the mean diameters of the in 3 leaves of 8 plants.
(B) Representative leaves of each genotype, showing symptoms of the disease observed 5 days after the inoculation with a drop of 6 µl of *Botrytis cinerea* spores (2,5 x 10⁴ conidia/ml).
(C) Resistance response of *ocp3 nahG, ocp3 npr1 and ocp3 pad4* double mutants to *Plectosphaerella cucumerina* compared to that of simple mutant genotypes and wild-type plants. The wound was measured by determining the mean diameters of the wound in 3 leaves of 8 plants. The data points show the mean size of the wound ± SE of the measurements.

### Figure 4. Effect of mutations related to JA and ET on the resistance response to diseases of ocp3 plants.

(A) Resistance response of *ocp3 coi1* and *ocp3 jin1* double mutants to *Botrytis cinerea* compared to that of simple mutant genotypes and wild-type plants. The plants were inoculated and the symptoms of the disease were evaluated as described in Figure 2, determining the mean diameters of the wound in 3 leaves of 8 plants.
(B) Resistance response of *ocp3 coi1* and *ocp3 jin1* double mutants to *Plectosphaerella cucumerina* compared to that of simple mutant genotypes and wild-type plants. The symptoms of the disease were evaluated by determining the mean diameters of the wound in 3 leaves of 8 plants.
(C) Representative leaves of each genotype, showing symptoms of the disease observed 7 days after the inoculation with a drop of 6 µl of *P. cucumerina* spores (5 x 10⁶ spores/ml). The data points show the mean size of the wound ± SE of the measurements.

### Figure 5. Positional cloning of ocp3 and reverse complementation

(A) 0.6 Mb region at the upper part of chromosome 5 with overlapping BACs flanked by the SSLP markers Nga249 and ca72 used for screening recombinations in 2200 chromosomes.
(B) Location of OCP3 in the sequenced BAC clone F2I11. OCP3 was placed between two SSLP markers comprising a 67.2 Kb region of BAC F2I11. The mentioned 19 genes included in this region are indicated.
(C) Structure of OCP3 exons/introns. The encoding regions are indicated with thick lines. The insert shows the nucleotide change and its influence on the protein sequence. The allele mutant is indicated under the wild-type sequence. The lower case letters mark nucleotide sequences at the start of exon 3. The G-to-A transition due to the mutagen is indicated in upper case letters in bold print. The deduced amino acid sequences are indicated in the single-letter code with upper case letters under each nucleotide triplet and the bold letters mark the amino acid changes (from Ala to Thr) in the protein sequences.
(D) Resistance response of transgenic *ocp3* plants (line 2AT) stably transformed with a 3.2 Kb genomic DNA sequence including the entire At5g11270 gene and comparison with the resistance response observed in the wild-type plants and in the *ocp3* mutant. The plants were inoculated as described in Figure 2 with B. *cinerea* (at the right-hand side) and P. *cucumerina* (at the left-hand side) and the symptoms of the disease were evaluated by determining the mean diameters of the wound in 3 leaves of 8 plants. The data points show the mean size of the wound ± SE of the measurements.
(E) Histochemical staining of the GUS activity directed by the Ep5C promoter in completely expanded rosette leaves obtained from ocp3 plants (at the left-hand side) and from transgenic ocp3 plants transformed with the At5g11270 gene (line 2AT) (at the right-hand side).

### Figure 6. Expression of OCP3 and marker genes of defense response after the infection with P. cucumerina

RT-PCR analysis of leaf tissue infected with P. *cucumerina.* Wild-type plants were inoculated by spraying with a suspension of 10⁵ spores/ml, and the tissue was frozen for RNA extraction. The numbers indicate the hours after the inoculation. The gels of the lower part show the RT-PCR for the internal elF4α gene used as a load control. The experiment was repeated twice, similar results being obtained.

### Figure 7. Analysis of OCP3 and ocp3 cDNA

(A) Diagram of the structure of OCP3 exons/introns. The exons are indicated with thick lines. The insert indicates the nucleotide sequence in the splice junction exon 3. The lower case letters mark intron sequences, the upper case letters mark exon sequences, the bold upper case letters indicate amino acids and the arrows indicate the corresponding substitution of nucleotides and of amino acids. The arrows at the upper part of the schematized gene indicate the different position of the primers used in the RT-PCR experiments. PfullD1 is located at the start of exon 1; pfullR1 is located at the end of exon 4; pD1 is located at the end of exon 2; pD2 is located at the start of exon 3 and pR1 is located in the middle of exon 3. D indicates the direct orientation (from 5' to 3') whereas R indicates the reverse orientation (from 3' to 5').
(B) Electrophoresis in agarose gel of RT-PCR products obtained when mRNA from wild-type plants (wt) and *ocp3* and a different combination of primers is used. The reduction of the molecular weight and thus, the faster migration of the amplified band from *ocp3* plants with pD1+pR1 primers compared to that obtained from wild-type plants should be observed. The absence of the amplified DNA product when pD2+pR1 primers and mRNA transcribed with reverse transcriptase from *ocp3* plants, but no from wild-type plants, should also be observed. The absence of amplified product indicates a lack of recognition by one of the two primers in the cDNA template generated from *ocp3* plants. The experiment was repeated several times with mRNA derived from 4 different *ocp3* and wild-type plants.
(C) Nucleotides sequence of and amino acid sequence derived from cDNA clones derived from mRNA isolated from wild-type (OCP3) and mutant (*ocp3*) plants. The reversely transcribed products were amplified with the pfullD1 and pfullR1 primers and the two chains were completely sequenced. The internal deletion of 36 nucleotides in all the sequenced *ocp3* cDNAs should be observed. The nucleotide sequence common to all the cDNAs derived from *ocp3* and OCP3 is underlined. The internal deletion in the *ocp3* cDNA affects the derived amino acid sequence and causes a phase shift generating a premature termination codon in the *ocp3* protein. The bold upper case letters mark amino acids and the asterisks indicate a termination codon. The arrow indicates the presence and position of the nucleotide (G) in OCP3 cDNA, producing the *ocp3* phenotype if it is mutated. The results were reproduced several times with mRNA derived from different wild-type and *ocp3* plants and in different stages of growth.

### Figure 8. Protein OCP3 and comparison with other proteins containing Arabidopsis homeodomains.

(A) Expected amino acid sequence of OCP3. The homeodomain is shown in bold. The two identification signals conserved for the nuclear localization are underlined. The acid domain is shown in italics and the domain interacting with the nuclear protein (LxxLL), included within this acid region, is underlined.
(B) Expected protein structure of OCP3. The relative position of the nuclear localization signals (NLS), of the domain of interaction with the nuclear protein (LxxLL), of the acid domain and of the homeodomain (HD) is indicated.
(C) Alignment of sequences showing the OCP3 C-terminal amino acid sequence with the homeodomain of different homeobox *Arabidopsis* genes including the members of the KN and HD-Zip family. The asterisk above the alignments corresponds to amino acid positions in the HD which are very conserved in all organisms and define the identification signal of the homeodomain. The black shading indicates amino acids conserved in all the entries, and the gray shading indicates amino acids with very similar physical and chemical characteristics.

### DETAILED EXPLANATION OF AN EMBODIMENT

### Plants, growth conditions and treatments

*Arabidopsis thaliana* plants were cultured in substrate or in plates containing Murashige and Skoog (MS) medium, as previously described (Mayda et al., 2000). The ocp3 mutant was isolated in an investigation of constitutive expressers of the Ep5C-GUS indicator gene in Columbia transgenic plants (Col-0) mutated with ethyl methanesulfonate (EMS), as previously described for another mutant (Mayda et al., 2000). The ocp3 mutant line used in these experiments was subjected three times to backcrossing with the wild-type parent line. The plants were cultured in a growth chamber at 20-22°C, with a relative moisture of 85%, and 100 µEm⁻² sec⁻¹ of fluorescent illumination, in a 14 hour light and 10 hour dark cycle.

Completely expanded leaves of four week-old plants were used for all the experiments (unless otherwise indicated). Staining was performed to determine the presence of H₂O₂ through the 3,3'-diaminobenzidine (DAB) uptake method, as previously described (Thordal-Christensen et al, 1997). Staining was performed to determine the presence of GUS activity as previously described (Mayda et al, 2000).

### Infection with pathogens

*Pseudomonas syringae* pv. *tomato* DC3000 (P.s. tomato *DC3000)* was cultured and prepared for the inoculation as previously described (Mayda et al. 2000). The density of the bacteria populations was determined by culturing serial dilutions in King's B agar medium supplemented with rifampicin (50 µg/ml) at 28°C and by counting the colony forming units. The data was presented as means and standard deviations of the logarithm (cfu/crm²) of at least six replicas. Three week-old plants were sprayed with a *P. parasitica* conidial suspension (10⁵ conidiospores ml⁻¹ of running water) as previously described (Mayda et al, 2000) for the resistance to *Peronospora parasitica* experiments. The density of the spores in the seedlings (seven pots per treatment, each pot treated separately) was evaluated on the seventh day using a hemocytometer. As an alternative, the leaf samples were stained with lactophenol-Trypan blue on different days after the inoculation and were examined under the microscope as previously described (Mayda et al, 2000). Three week-old seedlings were transplanted in individual pots and were cultured at 22°C during the day/18°C at night, with twelve hours of light every 24 hours for the resistance to *Plectosporium* and *Botrytis.* When the plants were six weeks old they were inoculated by applying drops of 6 ml drops of *Plectosphaerella cucumerina* (5 x 10⁶ spores ml⁻¹) or *Botrytis cinerea* (2.5 x 10⁴ conidium ml⁻¹) spore suspension to 3 completely expanded leaves per plant. P. *cucumerina* was isolated from naturally infected *Arabidopsis (Landsberg erecta* access) and was cultured in 19.5 g/l of potato-dextrose agar (Difco, Detroit) at room temperature for 2 weeks before collecting the spores and suspending them in 10 mM MgSO₄. B. *cinerea* (BMM1 strain isolated from *Pelargonium zonale)* was cultured in 19.5 g/l of potato-dextrose agar (Difco, Detroit) at 20°C for 10 days. The conidia were collected and resuspended in sterile PDS (12 g l⁻¹, Difco). The plants were maintained with a relative moisture of 100% and the symptoms of the disease were evaluated from 4 to 10 days after the inoculation, determining the mean diameter of the wound in 3 leaves from 5 plants.

### Genetic analysis

Crosses were performed by emasculating unopened buds and using the pistils as pollen receptors. Backcrossings were performed with the parent transgenic line using Ep5C-GUS plants as pollen donors. Reciprocal crosses were also performed. F1 and F2 plants were cultured in MS plates and assayed with regard to GUS activity. The segregation of the phenotype in the F2 generation was analyzed with a chi-square test to check the goodness of fit.

### PCR-based Mapping

An *ocp3* plant (in Columbia as a basis) was crossed with *Landsberg erecta* and used for mapping the progeny which segregated *ocp3* homozygotic mutants after self-pollination. Seedlings of the F2 population where selected for DNA extraction, and the recombinant seedlings were identified using single sequence length polymorphism (SSLP) markers according to the protocol described by Bell and Ecker (1994) and with new markers as indicated on the *Arabidopsis* database webpage (http://genome-www.stanford.edu).

### Generation of Double Mutants

The mutant alleles used throughout this invention were *npr1-7* (Cao et al., 1997), *pad4-1* (Zhou et al., 1998), *coi1-1* (Xie et al., 1998), *ein2-5* (Alonso et al., 1999) and *jin1-1* (Lorenzo et al., 2004). Transgenic plants (in the Columbia ecotype) which expressed the bacterial nahG gene have been described (Reuber et al. 1998). The double mutants *ocp3 npr1, ocp3 pad4, ocp3 coi1, ocp3 ein2, ocp3 jin1* and *ocp3 nahG* double mutants were generated using *ocp3* as a pollen receptor. The homozygosity of the loci was confirmed using molecular markers for each of the alleles in segregation populations. All the double mutants were confirmed in the F3 generation, except the *ocp3 coi1* plants which were sterile and could not be propagated in heterozygosity for coi1. For the double mutant that contained *ein2-5,* the F2 seeds were cultured in plates with MS that contained 20 µM 1-aminocyclopropane-1-carboxylic (ACC) acid and were placed in a growth chamber. After three days in the dark, the seedlings were evaluated with regard to the presence or absence of the ethylene (ET)-induced triple response (Guzman and Ecker 1990). The *ein2* mutant, which was insensitive to ET, did not have the triple response. F2 plants lacking the triple response were collected and transferred to the substrate to evaluate the homozygosity for *ocp3.*

### Genomic DNA and cDNA Cloning

The genome sequence was used as a basis for the cDNA cloning and genomic clones. Poly(A*) RNA was isolated from different wild-type and *ocp3* plants and were transcribed with reverse transcriptase using oligo-primers (dT) as described (Mayda et al., 1999). They were used as templates for amplifying OCP3 and *ocp3* cDNA using different combinations of sense and anti-sense primers with gene specificity:
pfuIId1 (5'-GAATTCATGATAAAAGCCATGG-5'),
pfuIIR1 (5'-GTTAACTCTAGATCTTTCCGGAG-5'),
pD1 (5'-GGTGATGTTGATGTTGATGTTG-3'),
pR1 (5'-CTTAGGTTCGACCACAACATCTTCAG-5') and
pD2 (5'-ATCTGGCAGCTGAGGTTTGTCTTG-5').

### Reverse Complementation

The OCP3 genomic region was amplified by PCR using primers with gene specificity designed to include the 1.5 Kb upstream region of the initiation codon and a part of region 3' after the termination codon. The sequences of the advance and reverse genomic primers of OCP3 used were:
(5'-GAGATTGGAACGTGGGTCGACTTTAG-3) and
(5'-TTCCTGAATTCATACTTTATCATAG-3'), respectively.

A 3.2 Kb genomic fragment which contained the wild-type At5g11270 gene was obtained by PCR using primers and was cloned into pCAMBIA1300 to produce the clone pCAMBIAOCP3 which was transferred to *Agrobacterium* and used to transform *ocp3* plants by the floral immersion method (Bechtold, N, Ellis, J. and Pelletier, G. (1993). In plant Agrobacterium-mediated gene transfer by infiltration of adult Arabidopsis thaliana plants. C. R. Acad. Sd. Paris Life Sci. 316, 1194-1199)

### Expression Analysis

To analyze the level of gene expression by reverse transcriptase-mediated PCR, total RNA samples were prepared from leaf tissue using the Totally RNA kit of Ambion (Austin, TX). Reverse transcription was performed using the RT kit for PCR of Clontech (Palo Alto, CA).

The series of oligonucleotide primers (50 pmol each) used for amplifying OCP3 were: OCP3PCR1 (5'-GCTTAAAAGACTGGCTTATGCATTG-3')/OCP3PCR2 (5'-GCTTTGGAGCGGGTCACGAAG-3').

The primers used for amplifying PDF1.2 were PDF1.2PCR1 (5'-ATGGCTAAGTTTGCTTCCAT-3')/PDF1.2PCR2 (5'-ACATGGGACGTAACAGATAC-3').

The primers used for amplifying PR1 were PR1PCR1 (5'-ATGAATTTTACTGGCTATTC-3')/PR1PCR2 (5'-AACCCACATGTTCACGGCGGA-3').

## Claims

1. *Arabidopsis thaliana* gene referred to as OCP3, **characterized by** encoding a homeobox-type transcription factor and by its nucleotide sequence SEQ. ID. No. 1.

2. An OCP3 gene according to claim 1, **characterized by** being an active repressor of the resistance response to necrotrophic fungal pathogens.

3. An OCP3 gene according to the previous claims, **characterized in that** its cDNA encodes a protein with 354 amino acid residues of 39111D and a pI of 4.53.

4. An OCP3 gene according to the previous claims, **characterized in that** it has close to the C-terminal end a homeodomain of 60 amino acids in positions 284 to 344.

5. An OCP3 gene according to the previous claims, **characterized in that** it has two canonic bipartite nuclear localization signals:
a) RK-(X)₁₀-KKNKKK in positions 64-81, and
b) KK- (X)₁₀-RRSKR in positions 294-310, the latter being within the homeodomain.

6. An OCP3 gene according to the previous claims, **characterized in that** it has an extended region rich in acid residues in positions 84-181.

7. An OCP3 gene according to the previous claims, **characterized in that** has a canonic LxxLL motif in positions 101-105, which is a typical sequence aiding the interaction of different transcription co-activators with nuclear receptors.

8. An OCP3 gene according to the previous claims, **characterized in that** it acts as a negative regulator of the jasmonic acid-mediated defense response to necrotrophic fungal pathogens.

9. An OCP3 gene according to the previous claim, **characterized in that** it acts as a COI1-dependent and jasmonic acid-mediated specific transcription factor of the plant cell signal transduction pathway.

10. A recessive mutation in the OCP3 gene, **characterized by** conferring greater resistance to ocp3 mutant plants to necrotrophic fungal pathogens.

11. An ocp3 recessive mutation according to claim 10, **characterized by** conferring constitutive expression of the PDF1.2 gene encoding a defense protein in the jasmonic acid-mediated defense response of plants.

12. An ocp3 recessive mutation according to claim 11, **characterized in that** the PDF1.2 gene is used as a marker of the jasmonic acid-mediated physiological/molecular response of plants.

13. An ocp3 recessive mutation according to claims 10, 11 and 12, **characterized in that** its cDNA has an internal deletion corresponding to the first 36 nucleotides of exon III.

14. An ocp3 recessive mutation according to claim 13, **characterized in that** it produces a truncated protein with 210 amino acid residues instead of the 354 amino acid residues of OCP3.

15. The use of the OCP3 gene in plant species of agricultural interest for obtaining transgenic plants resistant to necrotrophic fungal pathogens.

16. Transgenic plants **characterized by** incorporating the OCP3 gene according to the previous claims, wherein the incorporated OCP3 gene has been genetically manipulated such that the expression of such gene is repressed or altered, or its function as a transcription factor is, conferring said plants greater resistance to necrotrophic fungal pathogens.
